# FASCICULE DE BREVET EUROPEEN

(11) **EP 1 671 671 B1**
(45) Date de publication et mention de la délivrance du brevet: **05.08.2009**
(21) Numéro de dépôt: 05292244.0
(22) Date de dépôt: 25.10.2005
(51) Int. Cl.: A61N 1/375, A61L 27/30, A61L 29/10

(54) **Connecteur revetu de carbone pour disposifs médicaux**
Kohlenstoffbeschichtete Verbinder für medizinische Vorrichtungen
Carbon coated connectors for medical devices

(30) Priorité: 25.10.2004 FR 0411331
(43) Date de publication de la demande: 21.06.2006
(73) Titulaire: ELA MEDICAL, F-92541 Montrouge (FR)
(72) Inventeur: Ceron, Claudio, 10090 Castagneto PO (IT); Gaggini, Guido, 20128 Milano (IT)
(74) Mandataire: Dupuis-Latour, Dominique

(56) Documents cités:
- DE-A1- 3 116 040
- FR-A- 2 654 345
- FR-A- 2 765 486
- GB-A- 2 331 998
- US-A- 4 220 386
- US-A- 4 612 100
- US-B1- 6 468 244

## Description

L'invention concerne les connecteurs pour "dispositifs médicaux" tels que définis par la Directive 93/42/CE du 14 juin 1993 du Conseil des communautés européennes, et notamment ― mais de manière non limitative ― aux "dispositifs médicaux implantables actifs" définis par la Directive 90/385/CE du 20 juin 1990.
L'invention s'applique avantageusement aux dispositifs actifs tels que prothèses cardiaques de stimulation, défibrillation et/ou cardioversion, mais elle peut aussi bien être appliquée de façon beaucoup plus générale à une très grande variété de dispositifs tels qu'appareils neurologiques, pompes ou chambres de diffusion de substances médicamenteuses, implants cochléaires, capteurs biologiques implantés, etc.
Ces dispositifs sont constitués d'un ensemble formé d'un boîtier et d'une ou plusieurs sondes mécaniquement reliées à ce générateur. La sonde peut être par exemple un cathéter pour la diffusion d'une substance médicamenteuse, donc avec une liaison purement mécanique au boîtier, ou bien être une sonde pourvue d'électrodes, nécessitant une liaison à la fois mécanique et électrique au générateur.
Ainsi, dans l'exemple d'un stimulateur cardiaque, le boîtier est constitué d'un générateur, contenant les divers circuits électroniques et la pile d'alimentation du dispositif, et d'une tête de connecteur solidaire du corps et pourvue d'un ou plusieurs logements femelles aptes à recevoir une fiche de connexion montée à l'extrémité proximale de la sonde. Le raccordement électrique et mécanique de la sonde au générateur est opéré par insertion de la fiche dans ce logement, manipulation réalisée par le chirurgien lors de l'implantation.
Une telle tête de connecteur est par example décrite dans le FR-A-2 765 486 (ELA Medical).
Le générateur doit être remplacé au bout de quelques années en raison de l'épuisement de la pile. L'intervention correspondante consiste à ouvrir la poche subcutanée contenant le générateur, à déconnecter de celui-ci la fiche de la sonde (la sonde restant en place), à insérer cette fiche dans le nouveau générateur puis replacer ce dernier dans la poche, qui sera refermée et suturée.
Concrètement, après plusieurs années, il est souvent difficile de séparer la fiche du générateur. En effet, au cours des années l'infiltration et le dépôt, de fluides corporels, de cellules mortes, de tissus fibreux, etc. a tendance à créer une adhésion de la fiche à la surface intérieure de la tête de connecteur. L'effort exercé sur la gaine de la sonde pour extraire la fiche afin de vaincre cette adhérence est tel qu'il provoque quelquefois une rupture de la gaine de la sonde, de sorte qu'il est nécessaire soit d'implanter une nouvelle sonde, soit de sectionner la partie terminale de celle-ci pour y rapporter et connecter une nouvelle fiche. Dans l'un ou l'autre cas, ces opérations sont longues et minutieuses, de sorte que, outre le risque accru pour le patient, la durée et le coût de l'intervention s'en trouvent sensiblement augmentés.
L'un des buts de l'invention est de remédier à cette difficulté, en proposant une structure de connecteur qui permette de disjoindre aisément la fiche de la tête de connecteur en dépit de l'infiltration et du dépôt de matières corporelles à l'interface fiche/tête de connecteur au fil des années.
La fiche, qui peut être insérée dans un logement d'une tête de connecteur du dispositif pour réaliser une liaison mécanique à ce dernier, est selon l'invention revêtue à sa surface extérieure d'une couche additionnelle de matériau antiadhérent en film de carbone.
De préférence, cette couche additionnelle s'étend essentiellement sur la longueur de la partie de fiche située à l'intérieur du logement après insertion de la fiche dans la tête de connecteur.
En variante, la couche peut être dépose sur la paroi intérieure du logement de la tête de connecteur.
La couche, qui est de préférence d'épaisseur uniforme inférieure à 1 µm, est notamment un film déposé par pulvérisation cathodique.

L'emploi de carbone pour des revêtements de parties de dispositifs implantables a déjà été décrit, dans des contextes différents.

Ainsi, le US-A-6 468 244 propose d'appliquer à la surface d'un ballonnet d'angioplastie un revêtement de fullerènes permettant d'induire des effets thérapeutiques sur le site d'intervention du ballonnet.

Le US-A-4 220 386 propose de réaliser le conducteur interne d'une sonde de stimulateur sous forme d'une tresse de monofilaments de fibre de carbone.

Le US-A-4 612 100 décrit un procédé pour déposer une couche de carbone vitreux à la surface d'une électrode de stimulation en contact avec le myocarde, pour éviter d'augmenter de façon excessive le seuil de stimulation.

Le GB-A-2 331 998 décrit un procédé de dépôt d'une couche de carbone sur les surfaces en contact de deux pièces (implants articulaires ou pièces de moteur automobile) en mouvement relatif sous forte charge mécanique.

Le FR-A-2 654 345 décrit l'application d'une couche de matière carbonée sur le boîtier d'un générateur implantable, pour contrôler la croissance des tissus autour de ce dernier au site d'implantation.

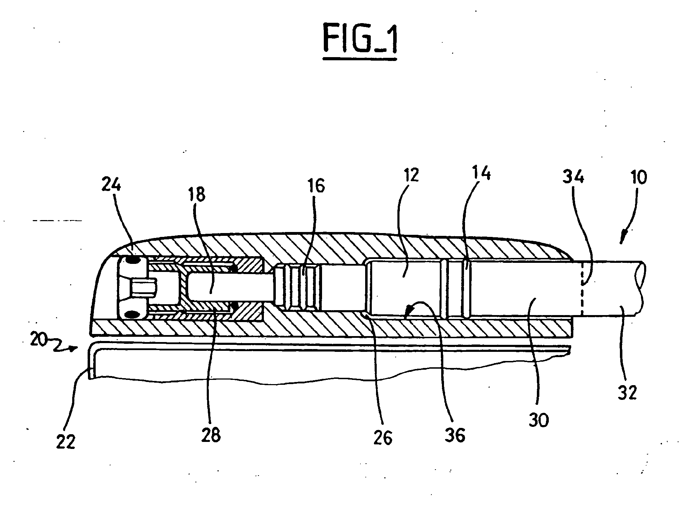

On va maintenant décrire un exemple de mise en oeuvre de l'invention, en référence à la figure unique annexée qui est une vue en coupe d'une tête de connecteur avec une fiche de sonde insérée à l'intérieur.

Sur la figure, la référence 10 désigne de façon générale l'extrémité proximale d'une sonde de dispositif implantable, par exemple une sonde endocardiaque de recueil/stimulation, de défibrillation, etc.

Cette sonde comporte une gaine isolante 12, généralement en matériau silicone, pourvue de reliefs annulaires tels que 14, 16 permettant d'assurer à la fois le positionnement axial précis et l'étanchéité côté sonde à l'enfichage. Dans l'exemple illustré, la sonde comporte un élément conducteur cylindrique d'extrémité 18 relié à l'électrode, ou à l'une des électrodes, de la sonde.
L'extrémité proximale de cette sonde est destinée à être reliée à un générateur 20, dont on a représenté la partie supérieure du boîtier 22 et la tête de connecteur 24. La tête de connecteur 24 comporte un logement cylindrique 26 dont les dimensions et la forme sont homologues de celles de la fiche de connexion, de manière à former un réceptacle pour cette dernière. La tête de connecteur comporte également un organe de connexion électrique 28 destiné à venir en contact avec l'élément conducteur 18 pour assurer la liaison de ce dernier aux circuits internes du générateur 20.
Selon l'invention, la fiche est revêtue à sa surface extérieure d'une couche additionnelle de matériau anti-adhérent, notamment un film de carbone déposé par pulvérisation cathodique.
Le dépôt de cette couche additionnelle est réalisé sur la région 30 de la fiche destinée à venir à l'intérieur du logement 26, en évitant de revêtir la partie apparente 32 qui restera à l'extérieur de la tête de connecteur. En d'autres termes, la frontière 34 entre les régions 30 et 32 devra se situer légèrement à l'extérieur de la tête de connecteur. En effet, le revêtement anti-adhérent ne doit pas gêner le chirurgien au moment où celui-ci exercera une traction sur la gaine de la sonde pour débrancher celle-ci du générateur : compte tenu de l'effort exercé, une prise satisfaisante va pouvoir être assurée, en évitant le glissement des doigts du chirurgien à l'endroit où il exerce son effort.
En variante ou en complément, le film anti-adhérent peut être déposé sur la surface de ia paroi intérieure 36 du logement 26.
Les US-A-5 370 684 et 5 387 247, tous deux au nom de Sorin Biomedica SpA, décrivent la manière de déposer par pulvérisation cathodique un film mince, submicronique, de carbone sur une prothèse implantée. On se réfèrera à ces deux documents pour plus de détails sur la technologie permettant de réaliser ce dépôt.
Ces documents illustrent le dépôt d'un film de carbone sur des implants tels que cathéters, valves cardiaques, etc. en polyuréthanne ou silicone. Toutefois, dans ces documents antérieurs, le film de carbone est choisi pour ses propriétés de biocompatibilité entre l'implant et l'environnement, afin de réduire les risques de rejet et/ou de dégradation de la prothèse, notamment sur les parties en contact avec un flux sanguin.
Dans la présente invention, le film de carbone est utilisé pour une autre de ses propriétés, qui est la réduction de l'adhérence entre deux pièces en contact, de manière à rendre la fiche "glissante" dans sa partie insérée dans la tête de connecteur.
Le choix du carbone présente en outre l'avantage d'une excellente biocompatibilité, permettant de garantir une stabilité élevée du revêtement appliqué sur le matériau polymère, même avec une couche de très faible épaisseur et même après avoir été en contact avec des matières biologiques pendant de nombreuses années.
Grâce à la présence de ce revêtement anti-adhérent, lorsque la sonde devra être retirée au moment d'un échange de générateur, même si des fluides corporels on pénétré dans la cavité du connecteur, la couche de carbone aura empêché le développement d'une adhérence entre le connecteur et le générateur, réduisant ainsi considérablement la force requise pour séparer ces deux éléments et, par voie de conséquence, le risque de rupture de la partie proximale de la sonde.

## Revendications

1. Une fiche de connexion montée à l'extrémité proximale (10) d'une sonde de dispositif médical implantable, cette fiche étant apte à être insérée dans un logement (26) d'une tête de connecteur (24) du dispositif pour réaliser une liaison mécanique à ce dernier,
fiche **caractérisée en ce qu'**elle est revêtue à sa surface extérieure d'un film de carbone antiadhérent.

2. La fiche de la revendication 1, où le film de carbone antiadhérent s'étend essentiellement sur la longueur de la partie (30) de fiche apte à se situer à l'intérieur dudit logement (26) après insertion de la fiche dans la tête de connecteur.

3. Un dispositif médical implantable (20), comprenant une tête de connecteur (24) avec un logement (26) apte à recevoir une fiche de connexion montée à l'extrémité proximale (10) d'une sonde pour réaliser une liaison mécanique à cette dernière,
dispositif **caractérisé en ce que** la paroi intérieure (36) du logement (26) est revêtue d'un film de carbone antiadhérent.

4. La fiche de la revendication 1 ou le dispositif de la revendication 3, où le film de carbone antiadhérent est d'épaisseur uniforme.

5. La fiche de la revendication 1 ou le dispositif de la revendication 3, où le film de carbone est un film déposé par pulvérisation cathodique.

6. La fiche de la revendication 1 ou le dispositif de la revendication 3, où l'épaisseur du film de carbone est inférieure à 1 µm.

## Claims

1. A connecting plug mounted on the proximal end (10 of a probe of an implantable medical device, wherein said plug is capable to be inserted into a cavity (26) arranged in a connector head (24) of the device to form a mechanical bond with the latter, wherein said connecting plug is coated on its external surface with an antiadhesive carbon film.

2. The connecting plug according to Claim 1, wherein the antiadhesive carbon film essentially extends along the length of plug part (30) capable of being situated inside said cavity (26) after plug insertion into the connector head.

3. Implantable medical device (20) comprising a connector head (24) with a cavity (26) capable of receiving a connecting plug mounted on the proximal end (10) of a probe to form a mechanical bond with the latter, wherein the interior wall (36) of the cavity (26) is coated with an antiadhesive carbon film.

4. The plug according to Claim 1 or the device according to Claim 3, wherein the antiadhesive carbon film has an uniform thickness.

5. The plug according to Claim 1 or the device according to Claim 3, wherein the carbon film is deposited by sputtering.

6. The plug according to Claim 1 or the device according to Claim 3, wherein the antiadhesive carbon film has an thickness less than 1 µm.

## Patentansprüche

1. Ein auf dem proximalen Ende (10) einer Sonde eines implantierbaren medizinischen Geräts montierter Verbindungsstecker, der geeignet ist, in einen Raum (26) eines Steckverbinderkopfs (24) des Geräts eingesetzt zu werden, um eine mechanische Verbindung mit dem letzteren zu bilden, **dadurch gekennzeichnet, dass** der Verbindungsstecker auf seiner Aussenoberfläche mit einem antiadhäsiven Kohlenstoff-film beschichtet ist.

2. Der Stecker nach Anspruch 1, wobei der antiadhäsive Kohlenstoff-film sich im wesentlichen auf der Länge jenes Teils (30) des Steckers erstreckt, welcher geeignet ist, sich im inneren des besagten Raums (26) nach dem Einsetzen des Steckers in das Steckverbinderkopf zu befinden.

3. Ein implantierbares, medizinisches Gerät (20, umfassend ein Steckverbinderkopf (24) mit einem Raum (26), der geeignet ist, einen auf dem proximalen Ende (10) einer Sonde montierten Verbindungsstecker aufzunehmen, um eine mechanische Verbindung mit dem letzteren zu bilden, **dadurch gekennzeichnet, dass** die Innenwand (36) des Raums (26) mit einem antiadhäsiven Kohlenstoff-film beschichet ist.

4. Der Stecker gemäss Anspruch 1 bzw. das Gerät gemäss Anspruch 3, wobei der antiadhäsiver Kohlenstoff-film eine gleichmässige Dicke besitzt.

5. Der Stecker gemäss Anspruch 1 bzw. das Gerät gemäss Anspruch 3, wobei der antiadhäsiver Kohlenstoff-film ein durch Kathodenzerstäubung aufgebrachter Film ist.

6. Der Stecker gemäss Anspruch 1 bzw. das Gerät gemäss Anspruch 3, wobei die Dicke des antiadhäsiven Kohlenstoff-films weniger als 1 µm beträgt.
